# EUROPÄISCHE PATENTSCHRIFT

(11) **EP 1 389 472 B1**
(45) Veröffentlichungstag und Bekanntmachung des Hinweises auf die Patenterteilung: **09.06.2010**
(21) Anmeldenummer: 03090236.5
(22) Anmeldetag: 28.07.2003
(51) Int. Cl.: A61L 31/16

(54) **Endovaskuläres Implantat mit aktiver Beschichtung**
Endovascular implant having an active coating
Implant endovasculaire avec un revêtement actif

(30) Priorität: 13.08.2002 DE 10237571
(43) Veröffentlichungstag der Anmeldung: 18.02.2004
(73) Patentinhaber: BIOTRONIK GmbH & Co. KG, 12359 Berlin (DE)
(72) Erfinder: Rohde, Roland, 31303 Burgdorf (DE); Dr. Sternberg, Katrin, 18057 Rostock (DE); Diener, Tobias, 91058 Erlangen (DE)
(74) Vertreter: Eisenführ, Speiser & Partner

(56) Entgegenhaltungen:
- EP-A- 1 236 478
- WO-A-01/62238
- WO-A-01/87376
- WO-A-02/32397
- WO-A-98/57631
- WO-A-99/48529
- US-A- 5 925 657
- BRIEGER DAVID ET AL: "Local drug delivery systems and prevention of restenosis" CARDIOVASCULAR RESEARCH, XX, XX, Bd. 35, Nr. 3, 1997, Seiten 405-413, XP002202664 ISSN: 0008-6363

## Beschreibung

Die Erfindung betrifft ein endovaskuläres Implantat, insbesondere einen Stent, mit zumindest einer abschnittsweisen aktiven Beschichtung sowie die Verwendung von PPARα-Agonisten und PPARδ-Agonisten zur lokalen Behandlung von Stenose oder Restenose.

Einer der häufigsten Todesursachen in Westeuropa und Nordamerika sind koronare Herzkrankheiten. Nach neuen Erkenntnissen sind vor allem entzündliche Prozesse treibende Kraft der Arteriosklerose. Initiiert wird der Prozess vermutlich durch die vermehrte Einlagerung von Lipoproteinen geringer Dichte (LDL-Partikel oder β-Lipoproteine) in die Intima der Gefäßwand. Nach dem Eindringen in die Intima werden die LDL-Partikel durch Oxidantien chemisch modifiziert. Die modifizierten LDL-Partikel veranlassen wiederum die Endothelzellen, die die inneren Gefäßwände auskleiden, das Immunsystem zu aktivieren. In der Folge treten Monocyten in die Intima ein und reifen zu Makrophagen heran. Im Zusammenspiel mit den ebenfalls eintretenden T-Zellen werden Entzündungsmediatoren, wie Immunbotenstoffe und proliferativ wirkende Substanzen, freigesetzt, und die Makrophagen beginnen die modifizierten LDL-Partikel aufzunehmen. Die sich bildenden Lipidläsionen aus T-Zellen und den mit LDL-Partikeln gefüllten Makrophagen, die aufgrund ihres Aussehens Schaumzellen genannt werden, stellen eine Frühform der arteriosklerotischen Plaque dar. Die Entzündungsreaktion in der Intima veranlasst durch entsprechende Entzündungsmediatoren glatte Muskelzellen der weiter außen liegenden Media der Gefäßwand bis unter die Endothelzellen zu wandern. Dort vermehren sie sich und bilden eine fibröse Deckschicht aus dem Faserprotein Kollagen, die den darunter liegenden Lipidkern aus Schaumzellen gegen die Blutbahn abgrenzt. Die dann vorliegenden tiefgreifenden strukturellen Veränderungen in der Gefäßwand werden zusammenfassend als Plaque bezeichnet.

Die arteriosklerotische Plaque expandiert zunächst relativ wenig in Richtung der Blutbahn, da diese sich kompensatorisch erweitern kann. Mit der Zeit kommt es jedoch zu einer Verengung des Blutkanals (Stenose), deren erste Anzeichen bei körperlicher Belastung auftreten. Die verengte Arterie kann sich dann nicht mehr ausreichend weiten, um das zu versorgenden Gewebe besser zu durchbluten. Ist eine Herzarterie betroffen, so klagt der Patient häufig über ein Druck- und Engegefühl hinter dem Brustbein (Angina pectoris). Bei anderen Arterien sind schmerzhafte Krämpfe ein häufig auftretendes Indiz für die Stenose.

Die Stenose kann letztendlich zu einem völligen Verschließen der Blutbahn führen (Herzinfarkt, Schlaganfall). Allein durch die Plaquebildung tritt dies nach neueren Untersuchungen jedoch nur in etwa 15 Prozent der Fälle auf. Vielmehr scheint der durch bestimmte Entzündungsmediatoren aus den Schaumzellen veranlasste allmähliche Abbau der fibrösen Deckschicht aus Kollagen ein entscheidender Zusatzfaktor zu sein. Reißt die fibröse Deckschicht auf, so kann der Lipidkem unmittelbar mit dem Blut in Kontakt kommen. Da in Folge der Entzündungsreaktion gleichzeitig Gewebsfaktoren (TF tissue factor) in den Schaumzellen produziert werden, die sehr potente Auslöser der Gerinnungskaskade sind, kann der sich bildende Blutpfropf das Blutgefäß verschließen.

Seit mehr als zwanzig Jahren sind nicht-operative Methoden zur Stenose-Behandlung etabliert, bei denen u.a. durch Ballondilatation (PTCA Perkutane Transluminale Coronare Angioplastie) das Blutgefäß wieder aufgeweitet wird. Mit dem Aufweiten des Blutgefäßes entstehen allerdings vereinzelt Verletzungen in der Gefäßwand, die zwar problemlos verheilen, jedoch in etwa einem Drittel der Fälle durch das ausgelöste Zellwachstum zu Wucherungen führen (Proliferation), die letztendlich zu einem erneuten Gefäßverschluss (Restenose) führen. Die Aufweitung beseitigt auch nicht die physiologischen Ursachen der Stenose, also die Veränderurigen in der Gefäßwand. Eine weitere Ursache der Restenose ist die Elastizität des gedehnten Blutgefäßes. Nach dem Entfernen des Ballons zieht sich das Blutgefäß übermäßig zusammen, so dass der Gefäßquerschnitt verringert wird (Obstruktion). Letzterer Effekt kann nur durch Plazierung eines Stents vermieden werden. Durch den Einsatz von Stents kann zwar ein optimaler Gefäßquerschnitt erreicht werden, allerdings führt der Einsatz von Stents ebenfalls zu kleinsten Verletzungen, die die Proliferation induzieren können und damit letztendlich die Restenose auslösen können.

Mittlerweile bestehen umfangreiche Erkenntnisse zum zellbiologischen Mechanismus und zu den auslösenden Faktoren der Stenose und Restenose. Die Restenose entsteht - wie bereits erläutert - als Reaktion der Gefäßwand auf die Dehnung der artherosklerotischen Plaque. Über komplexe Wirkmechanismen wird die lumengerichtete Migration und Proliferation der glatten Muskelzellen der Media und der Adventitia induziert (neointimale Hyperplasie). Unter Einfluss verschiedener Wachstumsfaktoren produzieren die glatten Muskelzellen eine Deckschicht aus Matrixproteinen (Elastin, Kollagen, Proteoglykane), deren ungesteuertes Wachstum allmählich zu einer Einengung des Lumens führen kann. Systematisch medikamentöse Therapieeinsätze sehen u.a. die orale Verabreichung von Calcium-Antagonisten, ACE-Hemmern, Antikoagulantien, Antiaggregantien, Fischölen, antiproliferativen Substanzen, antiinflammatorischen Substanzen und Serotonin-Antagonisten vor, signifikante Reduktionen der Restenoseraten wurden auf diesem Wege bisher jedoch nicht erreicht.

Das sogenannte Konzept des Local Drug Delivery (LDD) sieht vor, den oder die Wirkstoffe unmittelbar am Ort des Geschehens und begrenzt auf dieses Arial freizusetzen. Dazu wird im allgemeinen eine dem Blutgefäß zugewandte Oberfläche des endovaskulären Implantates, also insbesondere eines Stents, mit einer aktiven Beschichtung versehen. Die aktive Komponente der Beschichtung in Form eines therapeutischen Wirkstoffes kann direkt an der Oberfläche des Implantates gebunden oder in einem geeigneten Arzneistoffträger eingebettet sein. Im letzteren Fall wird durch Diffusion und gegebenenfalls allmählichen Abbau des biodegradierbaren Trägers der Wirkstoff freigesetzt

Als Wirkstoffe und Wirkstoffkombinationen wurden zahlreiche Präparate vorgeschlagen, jedoch ist der bisher in therapeutischen Versuchen nachgewiesene Effekt nur mäßig und die eingesetzten Pharmaka sind teils sehr kostenintensiv.

Seit längerem stehen PPAR-Agonisten als Wirkstoffe zur Behandlung der Typ-2-Diabetes und als Lipidsenker zur Verfügung. Unter der Bezeichnung Peroxisomproliferator aktivierte Rezeptoren (PPAR) wird eine Klasse von steroidhormonähnlichen nukleären Rezeptoren zusammengefasst. Derzeit sind drei PPAR-Isoformen, nämlich PPARα, PPARβ und PPARγ mit seinem Subtypen γ1 und γ2, bekannt. Die Bezeichnung PPARβ ist historisch bedingt. Dieser Rezeptor wurde erstmals am Krallenfrosch gefunden. Später fand man in höheren Tieren das entwicklungsgeschichtlich aus dem β-Rezeptor hervorgegangene PPARδ. Die Rezeptorsysteme lassen sich funktionell den Steroidhormon-Rezeptoren zuordnen und sind somit spezifische liganden-aktivierte Transkriptionsfaktoren, mit deren Hilfe Liganden (Steroidhormone, Peroxisom-Proliferatoren und viele andere) auf die Synthese von Proteinen Einfluss nehmen, wenn das entsprechende Gen responsiv ist. Bekannte körperfremde Peroxisom-Proliferatoren finden sich unter pharmazeutischen Wirkstoffen zur Behandlung von Diabetes und Hyperlipidämie sowie unter Insektiziden, Herbiziden, Fungiziden, Holzschutzmitteln, industriellen Schmierstoffen und anderen Xenobiotika.

Als Antidiabetika, insbesondere zur Behandlung der Insulinresistenz bei Typ-2-Diabetes, werden im zunehmenden Maße Glitazone, wie Pioglitazon und Rosiglitazon, eingesetzt. Die Wirkstoffgruppe der Glitazone weisen als gemeinsames funktionelle Gruppe einen Thiazolidin-2,4-dion-Rest auf. Derzeit wird davon ausgegangen, dass die insuiinseisitizer an nukleäre PPARγ-Rezeptoren binden und Bindung die Transkription von Genen, die an der Adipozytendifferenzierung beteiligt sind bewirkt. Ferner wurde eine verstärkte Expression von Lipoprotein-Lipasen, Fettsäuretransport-Enzymen und Acyl-CoA-Synthase beobachtet, die eine Senkung des Triglyceridspiegels und der freien Fettsäuren bewirken. Einhergehend mit weiteren Effekten führt die orale Applikation der Glitazone zu einer Verbesserung der Glukoseverwertung in Muskel- und Fettzellen.

Seit einigen Jahren werden Lipidsenker zur oralen Applikation als Arterioskleroseprophylaxe eingesetzt. Bekanntermaßen werden endogene Triglyceride in der Leber in Lipoproteine sehr niedriger Dichte (VLDL oder Prä-β-Lipoproteine) eingehüllt und in die Gefäßbahn sezerniert. Bestimmte Lipoprotein-Lipasen spalten aus den VLDL-Partikeln einen Teil der Triglyceride, wobei der Abbau zu Lipoproteinen niederer Dichte (LDL) führt. Die LDL-Partikel sind die Hauptcholesterinträger des Plasmas. Ihre Konzentration kann einerseits durch Zunahme von Sekretion und Abbau der triglyceridischen VLDL-Partikel und andererseits durch verminderten LDL-Abbau ansteigen. Der LDL-Abbau erfolgt intrazellular vornehmlich zur Synthese von Membranen, wobei zunächst durch oberflächlich auf der Zellmembran angeordnete Rezeptoren die LDL-Partikel in die Zelle eingeschleust werden. Bei chronischem Überangebot an LDL sinkt die Zahl der LDL-Rezeptoren an der Membranoberfläche, sodass LDL vermehrt im Plasma verbleibt und wie bereits erläutert in den Arterienwänden abgelagert und schließlich modifiziert wird.

Beim Abbau von VLDL bzw. LDL werden die als Lösungsvermittler dienenden Oberflächensubstanzen teilweise abgegeben. Diese können wiederum Fett umhüllen und die entstehenden Lipoproteinstrukturen haben ein höheres spezifisches Gewicht (HDL oder α-Lipoproteine). Die HDL-Partikel ermöglichen auf Grund ihres spezifischen Aufbaues die Bindung von überflüssigen Lipiden (Triglycerin oder Cholesterin) aus den Geweben, also auch beispielsweise auch aus der Arterienwand. Hohe HDL-Konzentrationen ermöglichen daher cholesterinbeladene Arterienwände zu reparieren. Eine gute lipidsenkende Therapie zielt somit auf die Senkung von VLDL und/oder LDL bzw. auf eine Erhöhung der HDL-Konzentration.

Zur Pharmakotherapie wird unter anderem der Einsatz des Wirkstoffs Clofibrat (2-(4-chlorphenoxy)-2-methylpropionsäureethylester) vorgeschlagen. Clofibrat, ein PPARα-Agonist, senkt die VLDL-Synthese in der Leber und aktiviert die Lipoprotein-Lipase. In der Folge wird der Triglyceridspiegel und weniger stark der Cholesterinspiegel des Plasmas gesenkt sowie unwesentlich die HDL-Konzentration gesteigert. Weitere Fibrate wie Etafibrat, Bezafibrat, Fenofibrat und Gemfibrozil werden teils als Monopräparat, teils als Kombinationspräparat im gleichen Sinne eingesetzt und erzielen mitunter deutliche Anstiege der HDL-Konzentration.

Die genannten PPAR-Agonisten werden bisher in der Praxis ausschließlich zur oralen Langzeitapplikation, insbesondere zur Behandlung des Typ-2-Diabetes und Prävention von Arteriosklerose, eingesetzt. Auf Grund der relativ hohen Dosis und der Langzeitanwendung ist allerdings mit unerwünschten Nebeneffekten zu rechnen. So wurde Studien zufolge bei der Langzeittherapie mit Clofibrat ein Anstieg von Nieren- und Gallenblasenerkrankungen und bei PPARγ-Agonisten über Ödembildung, Gewichtszunahme und Hepatotoxizität berichtet.

Die PPARs bilden nach neueren Studien Heterodimere mit einer weiteren Form nukleärer Rezeptoren, den 9-cis-Retinsäure-Rezeptoren (RXR). Die PPAR/RXR-Heterodimere binden an spezifische DNA-Sequenzen, die als Promotoren bestimmter Gene wirken, wie beispielsweise von Acyl-CoA-Oxidase (AOX) oder adipozyten Fettsäure-Bindeproteinen (aP2). Die Bindung eines Agonisten sowohl an PPAR als auch an RXR führt in der Regel zu einer Änderung des Expressionsniveaus von mRNA, die durch die Zielgene des Heterodimers codiert wird (Transaktivierung).

Das Zytostatikum Bexaroten wird zur Therapie des cutanem T-Zell-Lymphom (CTCL) eingesetzt. Der genaue Wirkungsmechanismus ist noch nicht bekannt. Vermutlich bindet Bexaroten als Agonist an spezifischen 9-cis-Retinsäure-Rezeptoren. In vitro hemmte Bexaroten das Wachstum entarteter hämatopoetischer Zellen. In vivo verhinderte es die Tumorregression beziehungsweise Neubildung.

Einer Studie zufolge soll Phytansäure ein natürlicher Rexinoid (RXR-Agonist) sein (McCarty M.F.; The chlorophyll metabolite phytanic acid is a natural rexinoid - potential for treatment and prevention of diabetes; Medical Hypotheses 56 (2001) 217-219).

Aus der nachveröffentlichten EP 1 236 478 ist eine aktive Beschichtung eines Stents mit wenigstens einem PPARγ-Agonisten bekannt. Als PPARγ-Agonistenwerden Glitazone genannt.

Aus der nachveröffentlichten WO 99/48529 ist ein Kombinationspräparat aus einem Retinoid und einem Glitazon bekannt, das zur systemischen Therapie von Restenose und Artherosklerose eingesetzt wird.

Aufgabe der vorliegenden Erfindung ist es, nicht in den gesamten Stoffwechsel des Patienten einzugreifen, sondern lediglich lokal therapeutische Ansätze zur Behandlung von Stenose oder Restenose bereitzustellen. Die endovaskulären Implantate sollen eine verbesserte Verträglichkeit, insbesondere mit Hinsicht auf etwaige inflammatorische und proliferative Prozesse in der Gewebsumgebung, gewährleisten.

Die Aufgabe wird durch das endovaskuläre Implantat, insbesondere einen Stent, mit einer zumindest abschnittsweisen aktiven Beschichtung mit den in Anspruch 1 genannten Merkmalen gelöst. Dadurch, dass die aktive Beschichtung als Wirkstoff einen oder eine Kombination von PPARα-Agonisten und PPARδ-Agonisten umfasst, lassen sich lokal, also nur in unmittelbarer Umgebung des Implantates, Stenose als auch Restenose wirkungsvoll behandeln bzw. verhindern. Die nur lokale Applikation in geringsten Wirkstoffmengen vermeidet Nebenwirkungen, wie sie beispielsweise bei der oralen Applikation von Antidiabetika und Lipidsenkern auftreten. Überraschenderweise zeigte sich, dass die Neointimaproliferation mit derartigen aktiven Beschichtungen deutlich gesenkt werden konnte. Offenbar führt die lokale Applikation der genannten PPAR-Agonisten im Bereich geschädigter arterieller Gefäßwände zu einer deutlichen Minderung inflammatorischer und proliferativer Prozesse.

Die Verwendung eines PPARα-Agonisten oder PPARδ-Agonisten oder eine Kombination aus beiden als Wirkstoff zur Herstellung einer zur lokalen Behandlung von Stenose oder Restenose geeigneten aktiven Beschichtung auf endovaskulären Implantaten stellt ein neues Indikationsgebiet für diese Wirkstoffgruppe dar und wird vollumfänglich beansprucht. Der Wirkstoff wird in einer pharmakologisch aktiven Applikationsform oder als Pro-Pharmaka und einer pharmakologisch wirksamen Dosierung auf der Oberfläche des Implantats bereitgestellt.

Vorzugsweise ist der Wirkstoff, wenn er PPARα-Agonisten umfasst, ein Fibrat, da diese Gruppe von PPARα-Agonisten bei lokaler Applikation im erfindungsgemäßem Sinne einem positiven therapeutischen Effekt auf die Restenose und Stenose zeigen. Insbesondere ist das Fibrat ein Wirkstoff aus der Gruppe Clofibrat, Etofibrat, Etofyllinclofibrat, Bezafibrat, Fenofibrat und Gemfibrozil. Gerade Clofibrat zeichnet sich durch seine einfache Verarbeitung, seinen geringen Preis und seine offenbar antiinflammatorische und antiproliferative Wirkung auf die Gewebsumgebung des Implantates aus.

Ferner ist bevorzugt, dass der Wirkstoff, wenn er zusätzlich PPARγ-Agonisten umfasst, ein Glitazon ist. Die PPARγ-Agonisten, insbesondere Ciglitazon, Pioglitazon, Rosiglitazon und Troglitazon, wirken offensichtlich antiinflammatorisch und antiproliferativ und senken damit u.a. die Restenosegefahr nach Implantation eines Stents.

Als RXR-Agonisten sind bevorzugt Bexaroten und Phytansäure.

Nach einer bevorzugten Variante der Erfindung werden die PPAR/-Agonisten in einen Arzneistoffträger eingebettet. Hierdurch lässt sich die Herstellung der beschichteten Implantate vereinfachen und die Freisetzung des Arzneistoffes steuern. Zudem kann ein unerwünschtes Abblättern des Wirkstoffes während der Implantation, insbesondere der Dilatation des Stents, wirkungsvoll unterdrückt werden. Es versteht sich von selbst, dass der Arzneistoffträger biokompatibel sein muss. Bevorzugt ist der Arzneistoffträger zusätzlich auch biodegradierbar, so dass über ein Abbauverhalten des Trägers eine gezielte Dosierung des Arzneistoffes möglich ist. In diesem Zusammenhang hat sich die Verwendung von Polylactiden, insbesondere Poly-L-Lactiden und deren Copolymere (z.B. Poly(L-Lactid-co-trimethylencarbonat), Poly(L-Lactid-co-D/L-Lactid)), Polydioxanon und Hyaluronsäure als besonders günstig erwiesen.

Eine Schichtdicke der aktiven Beschichtung liegt im Falle von Arzneistoffträgern mit eingebettetem Wirkstoff vorzugsweise zwischen 5 bis 30 µm, insbesondere zwischen 8 bis 15 µm. Eine Gewichtsmasse pro Implantat, also das Gewicht des Arzneistoffträgers plus Wirkstoff, liegt vorzugsweise im Bereich von 0,3 bis 2 mg, insbesondere 0,5 bis 1,5 mg, besonders bevorzugt 0,5 bis 1 mg. Mit den gewählten Bereichen lässt sich eine hohe lokale Wirkung erzielen, ohne dass es zu den gefürchteten Nebenwirkungen in Niere, Galle usw. kommen kann. Derartig dünne Beschichtungen neigen nicht zur Rissbildung und wirken demnach einem Abblättern bei mechanischer Beanspruchung (Stentdilatation) entgegen.

Wird ein biodegradierbarer Arzneistoffträger eingesetzt, so kann die Elutionscharakteristik insbesondere durch eine Variation des Vernetzungsgrades der Polymermatrix oder eine Variation des Polymerisationsgrades beeinflusst werden. Neben der Degradation des Trägers sind Diffusionsvorgänge für die Elution des Wirkstoffes maßgebend. Strukturelle Eigenschaften des Trägers (wie Kristallinität, Molekulargewicht, Verschlaufungsdichte) und des Wirkstoffs beeinflussen neben vielen weiteren Faktoren die Diffusionsgeschwindigkeit. Die Elutionscharakteristik einer derartigen aktiven Beschichtung wird vorzugsweise derart eingestellt, dass 10 bis 80 %, insbesondere 15 bis 70 %, besonders bevorzugt 15 bis 25 % des Wirkstoffes inner halb der ersten zwei Tage freigesetzt wird. Der Rest des verbleibenden Wirkstoffes soll - ebenfalls gesteuert über Diffusions- und Degradationsvorgänge - sukzessive innerhalb der ersten Monate abgegeben werden. Es hat sich überraschender Weise gezeigt, dass diese an sich relativ kurzen Zeiträume bereits eine wirkungsvolle Unterdrückung der neointimalen Proliferation ermöglichen.

Vorzugsweise werden die Fibrate in einer Dosis von 0,05 bis 1 mg, insbesondere 0,1 bis 0,75 mg, besonders bevorzugt 0,1 bis 0,3 mg, auf das endovaskuläre Implantat aufgebracht. Die Dosis von Glitazonen pro Implantat liegt bei vorzugsweise 0,01 bis 0,5 mg, insbesondere 0,02 bis 0,2 mg. Die RXR-Agonisten Bexaroten und Phytansäure werden vorzugsweise in Dosierungen von 5 bis 100 µg, insbesondere 10 bis 100 µg, eingesetzt. Die Dosis der Wirkstoffe ist so gering, dass selbst bei unterstelltem vollständigen Abtransport der Wirkstoffe durch das Blutplasma nicht mit einer für den Gesamtorganismus belastenden Dosis gerechnet werden muss. Lokal reicht dagegen die Dosierung aus, um den gewünschten Effekt auf die Restnoseprohylaxe zu erzielen.

Da die Diffusionsvorgänge ausgehend von der Implantatsoberfläche in die Intima und nachfolgend in die Media der Gefäßwand relativ langsam ablaufen, sollte das Implantat möglichst großflächig an seiner Außenseite mit der aktiven Beschichtung bedeckt sein. Die Aufbringung des Wirkstoffes bzw. des Wirkstoffes inklusive eines Arzneistoffträgers erfolgt-vorzugsweise mit Rotationszerstäubern, die einen fein verteilten Nebel aus kleinsten Schwebeteilchen erzeugen. Der Nebel benetzt oberflächlich kleinste Strukturen auf dem Implantat und wird anschließend durch Abblasen getrocknet. Dieser Vorgang kann beliebig wiederholt werden, bis die gewünschte Schichtdicke erreicht ist. Sofern gewünscht, können auf diese Weise auch mehrschichtige Systeme - beispielsweise für die Kombination von verschiedenen PPAR-Agonisten erzeugt werden, die aufeinanderfolgend aufgebracht werden. Ferner kann auf diese Weise ein Konzentrationsgradient in der Beschichtung erzeugt werden, so dass beispielsweise zu Beginn der Freisetzung eine erhöhte Menge des Wirkstoffes eluierbar ist, die dann sukzessiv abnimmt. Ferner ist eine Retardation der Wirkstofffreisetzung durch eine wirkstofffreie polymere Deckschicht, ein sog. Topcoat, denkbar.

Ferner ist es vorteilhaft, wenn ein Grundkörper des Implantates aus zumindest einem Metall oder zumindest einer Metalllegierung geformt ist. Vorteilhaft ist weiterhin, wenn das Metall oder die Metalllegierung zumindest teilweise biodegradierbar ist. Die biodegradierbare Metalllegierung kann insbesondere eine Magnesiumlegierung sein. Der Stent wird nach der biodegradierbaren Variante mit der Zeit vollständig abgebaut und damit verschwinden auch mögliche Auslöser für eine inflammatorische und proliferative Reaktion des umgebenden Gewebes.

Ein Stentdesign sollte vorzugsweise derart angepasst sein, dass ein möglichst großflächiger Kontakt zur Gefäßwand besteht. Dies begünstigt eine gleichmäßige Elution des Wirkstoff, die Untersuchungen zufolge im wesentlichen diffusionskontrolliert ist. Bereiche hoher mechanischer Verformbarkeit sind vorzugsweise bei der Beschichtung auszusparen, da hier die Gefahr des Abblätterns der Beschichtung erhöht ist. Alternativ oder in Ergänzung hierzu kann das Stentdesign derart vorgegeben werden, dass bei mechanischer Belastung, d.h. in der Regel bei der Dilatation des Stents, eine möglichst gleichmäßige Verteilung der auftretenden Kräfte über die gesamte Stentoberfläche gegeben ist. Auf diese Weise können lokale Überbeanspruchungen und damit' eine Rissbildung oder gar Abblättern der Beschichtung vermieden werden.

Ein sehr hohes Haftungsvermögen zeigt die aktive Beschichtung, wenn das Implantat eine passive Beschichtung aus amorphem Siliziumkarbid aufweist. Die polymere Beschichtung kann direkt auf die passive Beschichtung aufgebracht werden. Alternativ können Spacer oder Haftvermittlerschichten, die auf der passiven Beschichtung angebunden werden, zur weiteren Steigerung des Haftvermögens der polymeren Beschichtung vorgesehen sein. Auch ist eine Aktivierung der zu beschichtenden Oberfläche mittels Plasma oder mittels nasschemischer Verfahren denkbar.

Weitere bevorzugte Ausgestaltungen der Erfindung ergeben sich aus den übrigen in den Unteransprüchen genannten Merkmalen.

Die Erfindung wird nachfolgend anhand von Ausführungsbeispielen und anhand der zugehörigen Zeichnungen näher erläutert. Es zeigen:
- Figur 1: eine schematische Draufsicht auf einen Teilausschnitt eines endovasku- lären Implantats in Form eines Stents;
- Figur 2: eine Schnittansicht durch ein Strukturelement des Stents mit einer akti- ven Beschichtung und
- Figur 3: ein zur Figur 1 alternatives Stentdesign.

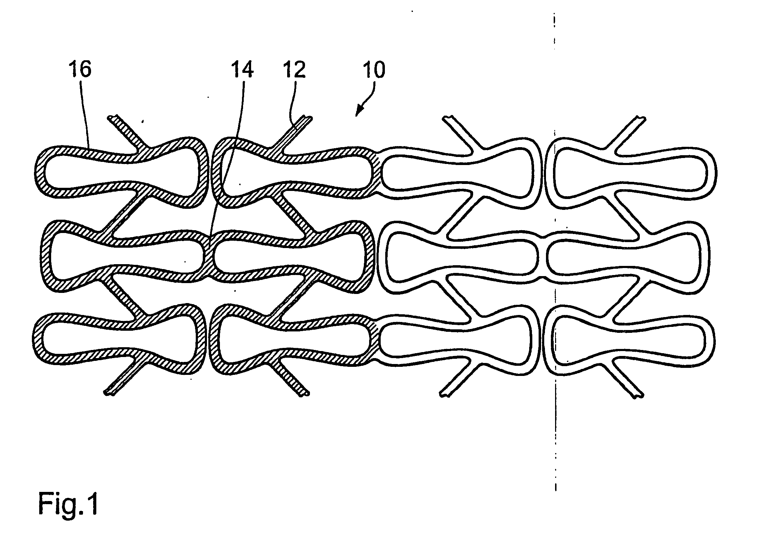

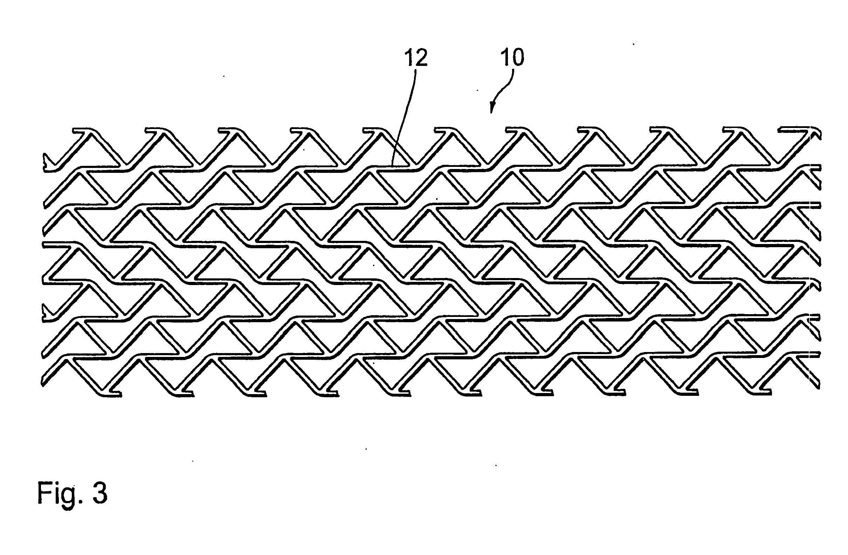

Die Figur 1 zeigt in eine schematische Draufsicht eines Teilausschnitts aus einem endovaskulären Implantat, hier in Form eines Stents 10. Der Stent 10 besteht aus einer Vielzahl von Strukturelementen 12, die - wie in diesem speziellen Beispiel dargestellt - ein netzartiges Muster um eine Längsachse des Stents 10 bilden. Derartige Stents sind seit langem in der Medizintechnik bekannt und können, was ihren strukturellen Aufbau angeht, im hohen Maße variieren. Von Bedeutung mit Sicht auf die vorliegende Erfindung ist, dass der Stent 10 eine nach außen gewandte, also nach Implantation zur Gefäßwand gerichtete Oberfläche 14 besitzt. Diese sollte im expandierten Zustand des Stents 10 eine möglichst große Flächenabdeckung besitzen, um eine gleichmäßige Wirkstoffabgabe zu ermöglichen. In Hinblick auf die mechanische Grundstruktur sind bei der Formgebung zu unterscheiden: Konzentration der Verformung auf wenige Bereiche oder gleichmäßige Verformung über die gesamte Grundstruktur. Im ersteren Falle sind die Strukturen derart ausgebildet, dass bei einer mechanischen Aufweitung des Stents nur Verformungen im Bereich von Fließgelenken konzentriert sind (so Beispielsweise in dem in Figur 1 gezeigten Stent 10). Die zweite Variante, bei der die Dilatation zu einer Verformung nahezu aller Strukturelemente 12 führt, ist exemplarisch in Figur 3 dargestellt. Es versteht sich von selbst, dass die Erfindung nicht auf die dargestellten Stentmuster beschränkt ist. Modifikationen des Stentdesign, die die Kontaktfläche vergrößern sind im allgemeinen bevorzugt, da bei wirkstoffbeladenen Beschichtungen eine gleichmäßigere Elution in die Gefäßwand ermöglicht wird. Ferner sind Bereiche hoher mechanischer Belastung, wie beispielsweise die Fließgelenke in Figur 1, entweder nicht zu beschichten oder es wird ein Stentdesign vorgegeben (z.B. das aus Figur 3), das die bei der Dilatation auftretenden Kräfte gleichmäßiger auf alle Strukturen des Stents verteilt. Damit soll eine Rissbildung oder ein Abblättern der Beschichtung infolge mechanischer Belastung vermieden werden.

Die Oberfläche 14 der Strukturelemente 12 ist mit einer aktiven Beschichtung 16-hier angedeutet durch die dunkelschraffierte Fläche - bedeckt. Die aktive Beschichtung 16 erstreckt sich über die gesamte Oberfläche 14 oder - wie hier dargestellt - nur über einen Teilabschnitt der Oberfläche 14. Die aktive Beschichtung 16 umfasst einen oder eine Kombination von PPAR-Agonisten, die in ihrer pharmakologisch aktiven Applikationsform auf die Oberfläche 14 der Strukturelemente 12 aufgebracht wurden und an dieser anhaften.

Unter dem Terminus pharmakologisch aktive Applikationsform" werden alle Eigenschaften des Wirkstoffes hinsichtlich Morphologie und Löslichkeit der Substanz bzw. ihrer Salze verstanden, die dazu beitragen, eine reproduzierbare Dosierung im Sinne einer therapeutischen Behandlung sicher zu stellen. So ist es häufig vorteilhaft amorphe, mikrokristalline Wirkstoffmodifikationen einzusetzen, da diese ein besonders schnelles und gleichmäßiges Elutionsverhalten zeigen.

Die aktive Beschichtung 16 beinhaltet als Wirkstoff Fibrate, und gegebenenfalls ergänzend Glitazone, Bexaroten und/oder Phytansäure. In Frage kommen insbesondere Fibrate aus der Gruppe Clofibrat, Etofibrat, Etofyllinclofibrat, Bezafibrat, Fenofibrat und Gemfibrozil und Glitazone aus der Gruppe Ciglitazon, Pioglitazon, Rosiglitazon und Troglitazon. Es hat sich jetzt überraschenderweise gezeigt, dass diese Wirkstoffe auch bei der lokalen Applikation zur Verhinderung der Restenose erfolgreich einsetzbar sind.

Die aktive Beschichtung 16 kann ferner einen Arzneistoffträger beinhalten, der biokompatibel ist und eine kontrollierte Freisetzung des Wirkstoffes erlaubt. Darüber hinaus dient der Arzneistoffträger auch zu einer verbesserten Anbindung der aktiven Beschichtung 16 an die Stentoberfläche 14, um ein Abblättern der aktiven Beschichtung 16 bei der Dilatation oder Einbringung des Stents 10 in ein arterielles Gefäß zu verhindern. Als Arzneistoffträger zeichnen sich insbesondere Hyaluronsäure und Polylactide inklusive deren Copolymere wie z.Bsp. Poly(L-Lactid-co-trimethylen carbonat) oder Poly(L-Lactid-co-D,L-Lactid) und auch Polydioxanon aus.

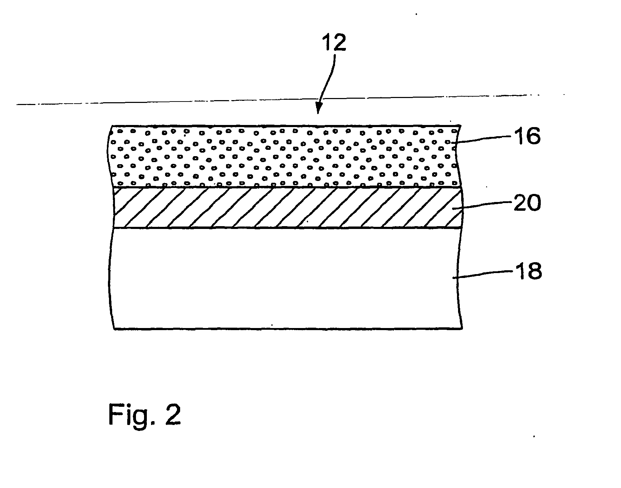

Eine besonders hohe Haftung auf der Oberfläche der Strukturelemente 12 kann dadurch erreicht werden, dass der Stent 10 an seiner Oberfläche 14 zusätzlich eine passive Beschichtung 20 aus amorphem Siliziumkarbid aufweist (siehe Figur 2). Die Herstellung derartiger Strukturen ist aus dem Stand der Technik, insbesondere aus dem Patent DE 44 29380 C1 der Anmelderin, auf dessen Offenbarung vollumfänglich verwiesen wird, bekannt und soll daher an dieser Stelle nicht näher erläutert werden. Es bleibt lediglich festzuhalten, dass ein Haftungsvermögen des aktiven Beschichtungsmaterials an der Stentoberfläche 14 mit einer solchen passiven Beschichtung 20 verbessert werden kann. Zudem mindert die passive Beschichtung 20 für sich allein bereits die neointimale Proliferation.

Eine weitere Verbesserung des Haftungsvermögens kann erzielt werden, wenn die Anbindung des polymeren Trägermaterials auf kovalentem Wege mittels geeigneter Spacer oder durch Aufbringung einer Haftvermittlerschicht erfolgt (hier nicht dargestellt). Grundzüge der Aktivierung der Siliziumkarbidoberfläche sind der DE 195 33682 A1 der Anmelderin zu entnehmen, auf deren Offenbarung hiermit vollumfänglich verwiesen wird. Als Spacer können photoreaktive Substanzen wie Benzophenonderivate eingesetzt werden, die nach reduktiver Kopplung an die Substratoberfläche und gegebenenfalls Entschützung funktionelle Anbindungsstellen für das Polymer bereitstellen. Eine wenige Nanometer dicke Haftvermittlerschicht lässt sich beispielsweise durch Silanisierung mit Epoxyalkylalkoxysilanen oder Epoxyalkylhalogensilanen und deren Derivaten erzielen. Anschließend wird das polymere Trägermaterial durch Physisorpition bzw. Chemisorption an die Haftvermittlerschicht angebunden. Das Vorgehen eignet sich besonders für die polymeren Trägermaterialien Polylactid und Hyaluronsäure.

In der Figur 2 ist eine Schnittansicht durch ein Strukturelement 12 des Stents 10 in einem beliebigen Bereich desselben dargestellt. Auf einem Grundkörper 18 mit vorgenannter passiver Beschichtung 20 aus amorphem Siliziumkarbid ist die aktive Beschichtung 16 aufgebracht. Der Grundkörper 18 kann aus Metall oder einer Metalllegierung geformt sein. Soll der gesamte Stent 10 biodegradierbar sein, so kann der Grundkörper 18 insbesondere auf Basis eines biodegradierbaren Metalls oder einer biodegradierbaren Metalllegierung hergestellt werden. Besonders geeignet ist eine biodegradierbare Magnesiumlegierung. Auch derartige Materialien sind bereits ausreichend im Stand der Technik beschrieben worden, so dass auf eine gesonderte Darstellung verzichtet wird. Es wird in diesem Zusammenhang insbesondere auf die Offenbarung der DE 198 56983 A1 der Anmelderin verwiesen.

Wenn der Arzneistoffträger biodegradierbar ist, kann die Elutionscharakteristik des Wirkstoffes durch Variation des Vernetzungsgrades der Polymermatrix oder eine Variation des Polymerisationsgrades beeinflusst werden. Das Vorgehen bietet sich insbesondere für die Arzneistoffträger Hyaluronsäure oder Polylactid an. Mit steigendem Vernetzungsgrad und steigender Molekularmasse des Polymers verlängert sich im allgemeinen auch der Zeitraum, in dem der Wirkstoff freigesetzt wird. Die Elutionscharakteristik einer derartigen aktiven Beschichtung wird vorzugsweise derart eingestellt, dass 10 bis 80 %, insbesondere 15 bis 70 %, besonders bevorzugt 15 bis 25 %, des Wirkstoffes innerhalb der ersten zwei Tage freigesetzt wird. Der Rest des verbleibenden Wirkstoffes soll - ebenfalls gesteuert über Diffusions- und Degradationsvorgänge - sukzessive innerhalb der ersten Monate abgegeben werden.

Die aktive Beschichtung 16 kann ferner im Aufbau strukturiert sein. Beispielsweise kann in den äußeren Bereichen der aktiven Beschichtung 16 ein geringerer Vemetzungsgrad als in den weiter innenliegenden Bereichen vorgesehen sein. Hierdurch kann der Abbau der aktiven Beschichtung 16 nach Implantation zunächst schneller erfolgen und bei gleichmäßiger Wirkstoffkonzentration in der aktiven Beschichtung 16 eine insgesamt höhere Anfangsdosis als in dem verbleibenden Zeitraum freigesetzt werden. Alternativ oder ergänzend kann dieser Effekt durch Vorgabe lokal unterschiedlicher Konzentrationen des Wirkstoffes in der aktiven Beschichtung 16 erreicht werden, indem beispielsweise die obersten Bereiche der Beschichtung 16 höhere Wirkstoffkonzentrationen aufweisen.

Die Herstellung der aktiven Beschichtung 16 wird mit Hilfe eines Rotationszerstäubers, der einen Nebel aus mikrofeinen Partikeln erzeugt, durchgeführt. Alternativ können auch Ultraschallzerstäuber eingesetzt werden. Die Beschichtung erfolgt schrittweise in zahlreichen Zyklen, die aus einem Benetzungsschritt des Stents im erzeugten Sprühnebel und einem anschließenden Trocknungsschritt des Niederschlags auf dem Stent durch Abblasen bestehen. Durch das mehrstufige Herstellungsverfahren lassen sich beliebige Schichtdicken und - sofern gewünscht - Konzentrationsgradienten des oder der Wirkstoffe in einzelnen Lagen der aktiven Beschichtung 16 erzeugen. Eine Sterilisation des Stents erfolgt durch Elektronenbeschuss, wobei ein teilweises Cracken der Polymerketten eines gegebenenfalls vorhandenen polymeren Träges bei hohen Molekulargewichten des Polymers in Kauf genommen werden kann. Die kinetische Energie der Elektronen liegt etwa im Bereich von 3,5 bis 6 MeV, insbesondere 4-5 MeV, da bei diesen Werten noch eine ausreichende Sterilisation bei nur geringer Eindringtiefe sichergestellt ist. Die Dosierung bewegt sich im Bereich von 15 bis 45 kGy, insbesondere von 15 bis 35 kGy pro Stent. Untersuchungen zeigten, dass keine oder nur eine minimale Minderung der biologischen Aktivität der Wirkstoffe durch das Sterilisationsverfahren erfolgt.

Die erzeugten Schichtdicken der aktiven Beschichtung 16 liegen in der Regel im Bereich von 5 bis 30 µm. Besonders günstig sind Schichtdicken im Bereich von 8 bis 15 µm, da hier eine weitestgehende Abdeckung der Oberfläche 14 des Stents 10 gewährleistet ist und noch nicht mit strukturellen Problemen wie Rissbildung und dergleichen gerechnet werden muss. Insgesamt werden pro endovaskulärem Implantat etwa 0,3 bis 2 mg, insbesondere 0,5 bis 1,5 mg an Beschichtungsmaterial aufgebracht, wenn die aktive Beschichtung 16 einen Arzneistoffträger beinhaltet. Eine Dosis des Wirkstoffes liegt bei Fibraten im Bereich von 0,05 bis 1 mg, insbesondere 0,1 bis 0,75 mg, und bei Glitazonen im Bereich von 0,01 bis 0,5 mg, insbesondere 0,2 bis 0,2 mg. Bexaroten und Phytansäure werden mit einer Dosis im Bereich von 5 bis 100 µg aufgebracht.

### Ausführungsbeispiel:

Als endovaskuläres Implantat wird ein handelsüblicher Stent, der unter dem Handelsnamen Lekton bei der Firma BIOTRONIK beziehbar ist, verwendet.

Der Stent wird in einen Rotationszerstäuber eingespannt. In einem Vorratsbehälter des Rotationszerstäubers wird eine Lösung von Poly-L-Lactid (beziehbar unter dem Handelnamen Resomer© L214 bei der Firma Boehringer Ingelheim) und Clofibrat in Chloroform bereitgestellt (Poly-L-Lactid-Konzentration: 7,5g/l). Der Massenanteil des Wirkstoffs Clofibrat zu der Masse des Arzneistoffträgers Poly-L-Lactid wird auf etwa 10% bis 50%, insbesondere 15% bis 40%, vorzugsweise 20% bis 30%, der Gesamtmasse eingestellt. Erprobt wurden Wirkstoffkonzentrationen von 15%, 30% und 40%.

Der Stent wird einseitig in 80 Zyklen von jeweils ca. 10 Sekunden Dauer mit einem durch den Rotationszerstäuber erzeugten fein verteilten Nebel benetzt. Der jeweiligen Benetzung folgt ein Trocknungsschritt durch Ablasen mit einer Dauer von ca. 12 Sekunden. Nach Beendigung der einseitigen Beschichtung erfolgt die Beschichtung der Rückseite des Stents gemäß dem gerade beschriebenen Vorgehen.

Nach dem Ende von insgesamt 160 Beschichtungszyklen wird der Stent entnommen und durch Elektronenbeschuss sterilisiert. Die Schichtdicke der aktiven Beschichtung liegt bei etwa 10 µm und die Masse der aktiven Beschichtung beträgt etwa 0,7 mg, woraus sich eine Wirkstoffmasse von etwa 140 µg pro Stent ergibt.

Der Stent wurde tierexperimentell am kardiovaskulären System des Schweins erprobt. Dazu wurde, der Stent alternierend in den Ramus interventricularis anterior (RIVA), Ramus circumflexus (RCX) und die rechte Koronararterie (RCA) des Herzens von 7 Schweinen eingepflanzt. Zu Vergleichszwecken wurde zeitgleich ein Blindversuch mit Stents ohne Beschichtung gestartet. Nach 4 Wochen wurden die Restenoseraten der Stents mit und ohne aktive Beschichtung durch Ausmessung der neointimale Proliferation mittels quantitativer Koronarangiographie bestimmt und verglichen. Es ergab sich eine signifikante Senkung der neointimalen Proliferation bei der Verwendung eines Stents mit aktiver Beschichtung.

## Patentansprüche

1. Endovaskuläres Implantat, insbesondere Stents, mit einer zumindest abschnittsweisen aktiven Beschichtung, **dadurch gekennzeichnet, dass** die aktive Beschichtung (16) als Wirkstoff einen PPARα-Agonisten oder PPARδ-Agonisten oder eine Kombination aus beiden zur lokalen Behandlung von Stenose und Restenose umfasst.

2. Implantat nach Anspruch 1, **dadurch gekennzeichnet, dass** der Wirkstoff ein Fibrat aus der Gruppe Clofibrat, Etofibrat, Etofyllinclofibrat, Bezafibrat, Fenofibrat und Gemfibrozil umfasst.

3. Implantat nach den Ansprüchen 1 oder 2, **dadurch gekennzeichnet, dass** die aktive Beschichtung (16) einen Arzneistoffträger aus der Gruppe Polylactid, Poly-L-Lactid und Hyaluronsäure umfasst.

4. Verwendung eines PPARα-Agonisten oder PPARδ-Agonisten oder eine Kombination aus beiden als Wirkstoff zur Herstellung einer zur lokalen Behandlung von Stenose oder Restenose geeigneten aktiven Beschichtung (16) auf einem endovaskulären Implantat.

## Claims

1. Endovascular implant, in particular stent, having an at least sectionally active coating, **characterized in that** the active coating (16) comprises a PPARα agonist or PPARδ agonist or a combination of the two for the local treatment of stenosis and restenosis as the drug.

2. Implant according to Claim 1, **characterized in that** the drug comprises a fibrate from the group clofibrate, etofibrate, etofyllinclofibrate, bezfibrate, fenofibrate, and gemfibrozil.

3. Implant according to Claim 1 or 2, **characterized in that** the active coating (16) comprises a pharmaceutical carrier from the group polylactide, poly-L-lactide, and hyaluronic acid.

4. Use of a PPARα agonist or PPARδ agonist or a combination of the two as a drug for producing an active coating (16), which is suitable for local treatment of stenosis or restenosis, on an endovascular implant.

## Revendications

1. Implant endovasculaire, en particulier stent, ayant un revêtement actif au moins par sections, **caractérisé par le fait que** le revêtement actif (16) comprend un agoniste de PPARα ou un agoniste de PPARδ ou une combinaison des deux, pour le traitement local d'une sténose et d'une resténose en tant que médicament.

2. Implant selon la revendication 1, **caractérisé par le fait que** le médicament comprend un fibrate provenant du groupe clofibrate, étofibrate, étofyllinclofibrate, bezfibrate, fénofibrate et gemfibrozil.

3. Implant selon la revendication 1 ou 2, **caractérisé par le fait que** le revêtement actif (16) comprend un support pharmaceutique provenant du groupe polylactide, poly-L-lactide et acide hyaluronique.

4. Utilisation d'un agoniste de PPARα ou d'un agoniste de PPARδ ou d'une combinaison des deux en tant que médicament pour produire un revêtement actif (16), qui est approprié pour le traitement local d'une sténose ou d'une resténose, sur un implant endovasculaire.
